## Europäisches Patentamt

## European Patent Office

## Office européen des brevets

(19)

(11) Numéro de publication: **0 258 136**
**B1**

(12)

# FASCICULE DE BREVET EUROPEEN

(45) Date de publication du fascicule du brevet:
27.06.90

(21) Numéro de dépôt: 87401889.8

(22) Date de dépôt: 14.08.87

(51) Int. Cl.⁵: **B23D 51/16,** B23D 47/12,
A61F 15/02

(54) Procédé et appareil pour couper des corps ou des matériaux durs sans couper des corps ou des matériaux plus tendres.

(30) Priorité: 20.08.86 FR 8611886

(43) Date de publication de la demande:
02.03.88 Bulletin 88/9

(45) Mention de la délivrance du brevet:
27.06.90 Bulletin 90/26

(84) Etats contractants désignés:
AT BE CH DE ES GB GR IT LI LU NL SE

(56) Documents cités:
DE-A- 1 931 890
DE-A- 2 430 064
DE-A- 3 408 189
US-A- 3 978 862

MACHINE DESIGN, vol. 35, décembre 1963, page 130;
"Oscillating saw cuts only solids"

(73) Titulaire: Tran Dinh, Can, 23, avenue Niel,
F-75017 Paris(FR)

(72) Inventeur: Tran Dinh, Can, 23, avenue Niel,
F-75017 Paris(FR)

(74) Mandataire: Lerner, François, 5, rue Jules Lefèbvre,
F-75009 Paris(FR)

ACTORUM AG

## Description

L'invention concerne un procédé et un appareil pour couper des corps ou des matériaux durs, mais ne coupant pas des corps ou matériaux plus tendres, tel que définis dans les préambules des revendications 1 et 4 (voir US-A 3 978 862), respectivement 5 (voir DE-A 1 931 890).

On connaît des appareils de coupe ou de découpe tels que des scies qui, animés d'un mouvement périodique, permettent de couper ou de débiter différents types de matériaux ou de corps, généralement sous l'impulsion d'un moteur électrique.

On peut noter en particulier dans ce type d'appareil, les scies circulaires et les scies sauteuses dans lesquelles l'organe de coupe est constitué respectivement par un disque denté entraîné en rotation autour d'un axe fixe et par une lame dentée entraînée dans un mouvement linéaire alternatif.

Toutefois, ce type de matériel pour utile qu'il soit, présente un certain nombre d'inconvénients, et notamment celui de couper la matière sur laquelle on applique ou on appuie l'organe de coupe quelle que soit la nature de cette matière : dure ou plus tendre. En d'autres termes, si l'utilisateur d'un tel matériel est maladroit ou inattentif ne serait-ce que pendant quelques instants, il risque de se blesser gravement si l'organe de coupe vient à toucher ou à entrer en contact avec une partie de son corps.

Le procédé et l'appareil de l'invention selon les revendications 1, 4 et 5 annule ou du moins diminue très largement de tels risques en coupant effectivement les matériaux ou les corps durs, sans couper ni endommager les matériaux ou corps plus tendres tels que la chair.

A cet effet, le procédé de l'invention est tel qu'on imprime à l'organe de coupe deux mouvements que l'on combine entre eux ; à savoir d'une part un premier mouvement périodique de relativement grande période orienté dans une direction de coupe déterminée, et d'autre part, dans cette même direction de coupe, un mouvement oscillatoire de plus petit période que celle dudit premier mouvement. De cette façon, on diminue de façon importante les risques ou les dangers liés à l'utilisation des appareils de coupe. En outre, on allonge la durée de vie de l'appareil dont l'organe de coupe s'use uniformément sans s'échauffer.

Dans un mode de réalisation de l'invention on prévoit, plus particulièrement, d'imprimer à l'organe de coupe d'une part un mouvement d'entraînement linéaire alternatif relativement lent et de grande amplitude orienté dans un axe de coupe déterminé et, d'autre part, dans ce même axe de coupe, un mouvement vibratoire plus rapide et de plus faible amplitude, et ce autour d'une succession de positions occupées par l'organe de coupe dans son mouvement linéaire alternatif.

Selon un autre mode de réalisation tout aussi intéressant, on prévoit, d'imprimer à l'organe de coupe d'une part un mouvement relativement lent d'entraînement en rotation autour d'un axe fixe et, d'autre part, toujours dans la direction de coupe, un mouvement vibratoire plus rapide et de relativement faible amplitude autour d'une succession de positions occupées par l'organe de coupe dans son mouvement de rotation.

On notera que, quel que soit le mode de réalisation retenu, les mouvements auxquels l'organe de coupe est soumis étant moins brutaux ou saccadés que sur les appareils à entraînement linéaire ou circulaire connus, la parti active de cet organe de coupe s'abime moins, ce qui facilite son entretien et augmente sa durée de vie.

Le procédé de l'invention s'appliquant, comme on l'a vu, en particulier aux scies sauteuses et circulaires, on notera, en ce qui concerne l'appareil de l'invention, qu'il comprend, outre un organe de coupe telle qu'une lame de scie, des moyens spécifiques d'entraînement appliqués à l'une ou l'autre de ces deux types de scies.

Ainsi, on obtient un appareil de coupe à la fois efficace et sans danger, alors qu'avec les appareils connus de ce type dans lesquels on imprimait à la lame un mouvement périodique relativement rapide et, suivant le cas, de grande ou de faible amplitude, on n'obtenait qu'une coupe dangereuse ou inéfficace pour les matériaux durs tels que le bois.

Le procédé et l'appareil de l'invention apparaîtront plus clairement de la description qui va suivre faite en référence aux dessins d'accompagnement dans lesquels :

- la figure 1 illustre schématiquement une vue intérieure de face d'un premier mode de réalisation de l'invention,
- la figure 2 illustre plus particulièrement les engrenages et les pièces de liaison de l'appareil illustré à la figure 1,
- la figure 3 présente une vue schématique intérieure de face d'une variante de réalisation de l'invention, et
- la figure 4 illustre plus particulièrement la position des différents engrenages et de l'arbre de commande de l'appareil illustré figure 3.

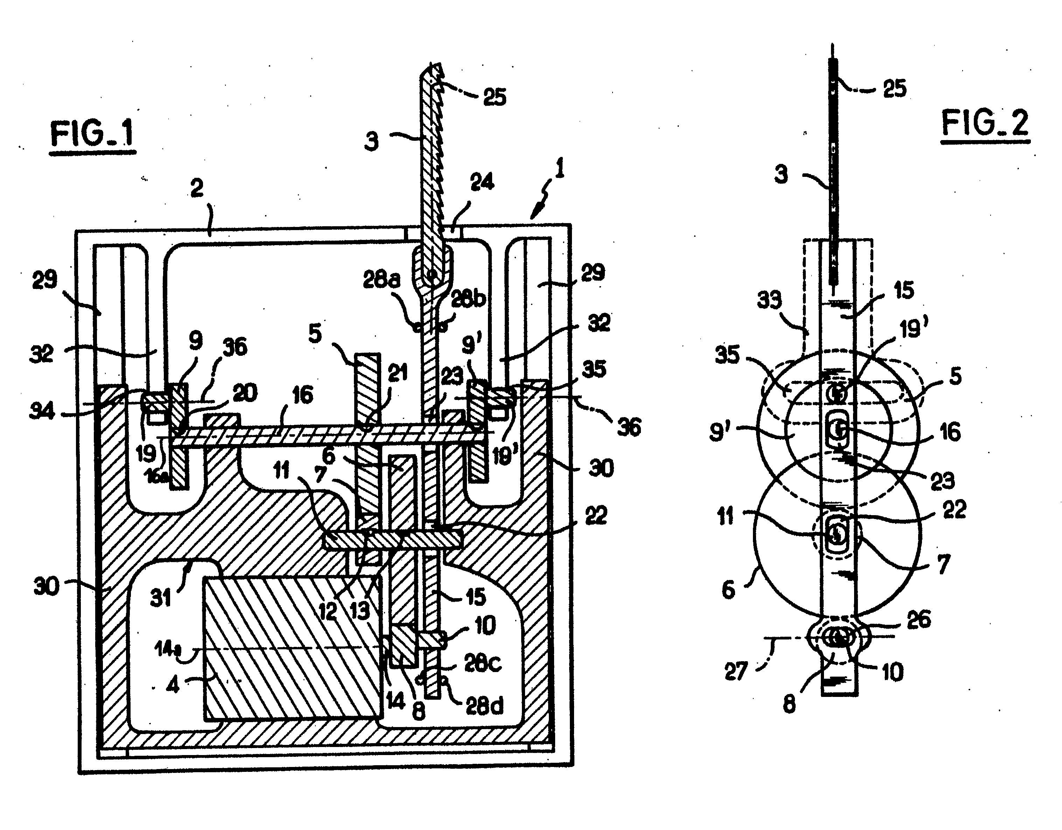

Si l'on se rapporte tout d'abord aux figures 1 et 2, on voit illustré un appareil de coupe conforme à l'invention du type "scie sauteuse".

L'appareil illustré et repéré 1, comprend un corps ou boîtier 2 à l'intérieur duquel sont disposés notamment un bâti support 31 mobile en translation par rapport au boîtier 2 sensiblement dans l'axe 25 d'une lame et divers moyens d'entraînement ou de liaison assurant à cette lame 3 dentée un déplacement en mouvements périodiques combinés de périodes différentes sous l'impulsion d'un moteur, de préférence électrique, 4 fixé au bâti 31.

Pour la clarté de la description, l'ensemble des moyens constitutifs de l'appareil seront décrits.

Le moteur 4 commande en rotation un arbre 14 de sortie qui traverse une roue d'engrenage 8 à laquelle il est lié. Cet arbre 14 se prolonge, vers son extrémité opposée au moteur, par un téton 10 qui est monté légèrement excentré par rapport à l'axe de rotation 14a de l'arbre 14 sur lequel il est calé et qui traverse une tige 15 liée rigidemnt à la lame, au niveau d'un orifice 26 allongé dans une direction 27 sensiblement perpendiculaire à l'axe 25 de la lame et à l'arbre 14.

Comme on le comprend, la lame 3 constitue l'organe de coupe de l'appareil, la tige 15 prolongeant cette lame dans son axe, vers l'intérieur de l'appareil.

Sur l'une quelconque des figures 1 ou 2, on remarque que 1 roue 8 est liée en engrènement avec une roue 6 transmettant le mouvement à une roue d'engrenage 7 par l'intermédiaire d'un arbre 11 et de clavettes 12, 13.

Cet arbre 11 est monté, parallèlement à l'arbre 14, fixe en translation mais libre de pivotement sur le bâti 31 vers ses extrémités opposées après avoir traversé, outre les roues 6 et 7, la tige 15 au niveau d'un orifice 22 allongé sensiblement parallèlement à la direction de l'axe 25 de la lame.

Quant à la roue 7, on notera qu'elle est liée en engrènement avec une roue 5 elle-même reliée, de part et d'autre, à deux roues 9, 9' par l'intermédiaire d'un arbre 16 et deux clavettes 20, 21.

L'arbre 16 s'étend parallèlement aux arbres 11 et 14, entre les roues 9, 9' en traversant notamment, outre la roue 5, la tige 15 au niveau d'un orifice 23 allongé sensiblement parallèlement à la direction de l'axe 25 de la lame.

Les roues 9, 9' sont munies chacune d'un téton excentrique 19, 19' qui s'étend parallèlement à l'axe 16a de l'arbre 16, chaque téton traversant une partie fixe 32, 33 du boîtier 2 au niveau d'orifices 34, 35 allongés sensiblement parallèlement à l'axe 27 déjà présenté, de sorte à guider le mouvement excentrique.

On notera que les tétons 19 et 19' sont montés plus excentrés par rapport à l'arbre 16 que l'est le téton 10 par rapport à l'arbre 4 de sortie moteur.

Les moyens principaux d'entraînement de la lame ayant été décrits, on notera que sur la figure 1 on a hachuré la partie mobile de l'appareil supportée par le bâti 31 ; les tétons excentriques 19 et 19' assurant la liaison entre le boîtier 2 fixe et l'ensemble des moyens constituant la partie mobile, en étant montés fixe en translation mais libre en rotation sur les parties 32 et 33 de de même boîtier. De façon à guider en déplacement cette partie mobile parallèlement à l'axe 25, on prévoit en particulier des glissières 29, 30 formées de façon complémentaires sur le bâti 31 et sur le boîtier 2. En outre, des galets 28a, 28b, 28c, 28d ou des moyens équivalents peuvent avantageusement guider la tige 15 de commande de la lame, laquelle s'étend vers l'extérieur du boîtier 2 au niveau d'un orifice débouchant 24.

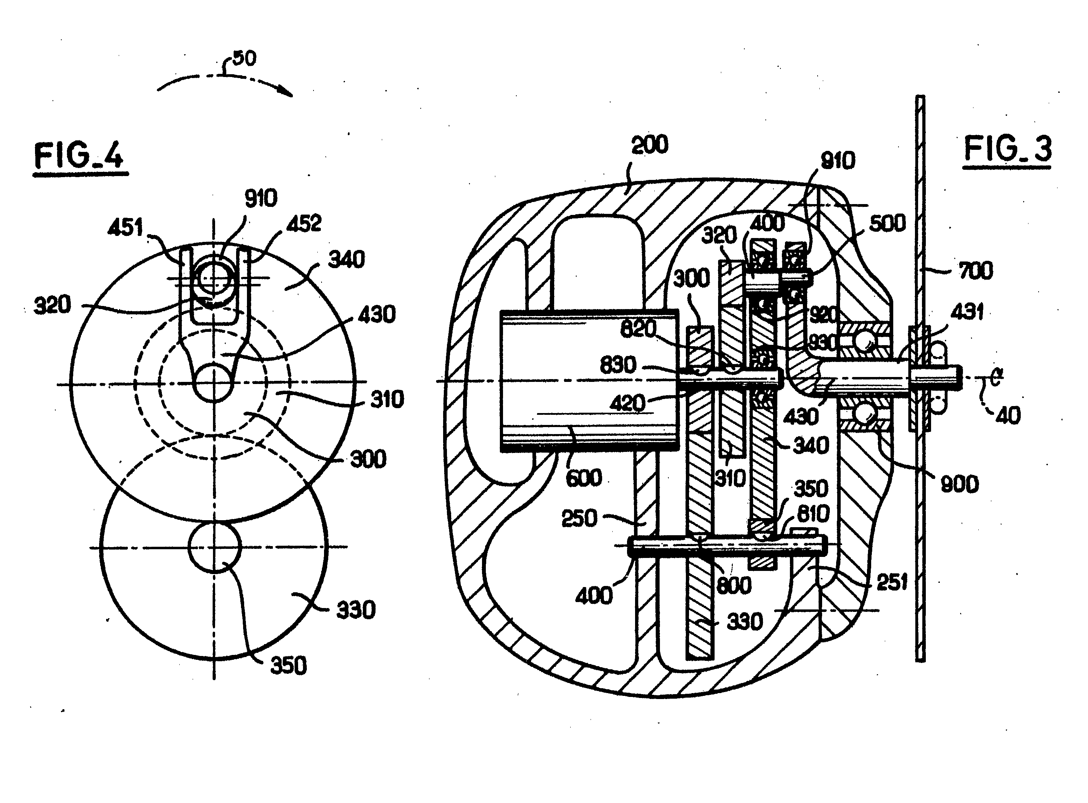

Si l'on se reporte maintenant aux figures 3 et 4, on voit illustré une variante de réalisation de l'appareil de l'invention présentant un matériel de type scie circulaire.

Comme dans l'appareil précédent, seule une partie des moyens d'entraînement ou de commande en mouvement de la lame fait l'objet de l'invention.

Toutefois, pour la clarté de la description, on décrira là encore, en détail, l'ensemble des moyens permettant de faire fonctionner cette scie circulaire.

L'appareil représenté comprend, comme celui illustré aux figures 1 et 2, un boîtier 200, diverses roues 300, 310-350, des arbres de liaison 400, 410...430, des clavettes 800, 810,...830, un moteur électrique d'entraînement en rotation 600, et un organe de coupe 700 du type disque denté.

Cet appareil est en outre muni de roulements à billes 900, 910...930 montés sur les arbres ou sur le téton excentrique 500.

Plus précisément, il apparaît des figures 3 et 4 que le moteur 600 qui est monté fixe sur le boîtier 200 de l'appareil commande la rotation d'un arbre de sortie 420, lequel transmet ce mouvement de rotation à deux roues d'engrènement 300 et 310 liées chacune audit arbre par une clavette 830, 820.

La roue 300 est par ailleurs liée en engrènement avec une roue 330 tandis que la roue 310 s'engène sur une roue 320 au niveau de laquelle est monté un arbre 400 qui s'étend parallèlement à l'arbre 420 et reçoit le téton excentrique 500 calé sur lui. Cet excentrique 500 s'étend notamment à l'intérieur d'un roulement à billes 910 monté sur un arbre 430, lequel à la forme générale d'un L̲ et débouche , vers l'extérieur du boîtier 200 suivant un axe 40 parallèle (ou confondu) avec celui du premier arbre 420, à travers un autre roulement à billes 900 monté sur une paroi de ce même boîtier.

L'arbre 430 assure au niveau de son extrémité débouchante 431 l'entraînement en rotation du disque de coupe 700.

Revenant sur les liaisons d'engrènement, on notera que la roue 330, déjà reliée à la roue 300, transmet le mouvement de rotation à une roue d'engrenage 350, et ce par l'intermédiaire d'un arbre 410 parallèle à l'arbre 420 et de clavettes 800 et 810 montées, respectivement, sur chacune des deux roues 330 et 350 ; l'arbre 410 étant lui-même monté libre de rotation au niveau de ses extrémités libres opposées sur des parties fixes 250, 251 du boîtier 200.

Si l'on se reporte plus particulièrement à la figure 3 il apparaît que la roue 350 est liée en engrènement avec une roue 340, laquelle roue 340 est traversée par les arbres 400 et 420 déjà présentés, ces arbres s'étendant à l'intérieur de roulement à billes repérés respectivement 920 et 930 montés sur ladite roue 340.

En se reportant maintenant à la figure 4 on remarque que l'arbre 430 d'entraînement du disque de coupe de l'appareil est, au niveau de son extrémité de liaison avec l'excentrique 500, formé en fourchette à deux dents 451, 452 entourant le roulement à billes 910 à l'intérieur duquel s'étend l'excentrique 500.

La description de deux exemples de variantes de l'appareil de l'invention ayant été faite, on va maintenant décrire leur fonctionnement selon lequel, pour couper des corps ou matériaux durs sans pour autant couper ni endommanger des corps ou matériaux plus tendres, on imprime à l'organe de coupe (lame ou disque, ou même éventuellement d'autres organes équivalents) deux mouvements périodiques de périodes différentes dirigés suivant une même direction de coupe et que l'on combine entre eux.

De façon plus spécifique, les pièces constitutives de la scie sauteuse illustrée aux figures 1 et 2 agissent de façon que l'on puisse imprimer à la lame 3, dans la direction de coupe matérialisée par l'axe 25, d'une part un mouvement linéaire d'entraînement alternatif relativement lent et de grande amplitude et, d'autre part, un mouvement vibratoire plus rapide et de plus faible amplitude, ce dernier mouvement s'effectuant autour d'une succession de positions occupées par la lame au cours de son mouvement linéaire alternatif.

Les deux mouvements combinés s'obtiennent comme suit.

a. Mouvement oscillatoire ou vibratoire de la lame de scie.

Le téton excentrique 10 calé sur l'arbre 14 lequel est commandé par le moteur 4, entraîne la tige 15 et donc la lame 3 en un mouvement rapid¨ de va et vient longitudinal et de faible amplitude sensiblement dans l'axe 25. On notera qu'à cet effet, la tige 15 est guidée par les deux arbres 11 et 16 qui s'étendent à travers les orifices allongés 22 et 23 et par les galets 28a, 28b, 28c et 28d.

b. Mouvement de va et vient de la lame et de l'ensemble de la partie mobile de l'appareil.

Par le système d'engrenages réducteurs 8-6, 7-5, les roues 9, 9' chacune avec son téton excentrique 19, 19', tournent lentement autour de l'axe 36 commun dans lequel s'étendent les deux tétons et imprime ainsi un mouvement relativement lent et ample de va et vient à l'ensemble de la partie mobile de l'appareil, donc en particulier à la lame 3, sensiblement dans l'axe 25.

En ce qui concerne la variante de réalisation de l'invention relative à la scie circulaire, on notera que, dans ce cas, au lieu d'imprimer un mouvement linéaire alternatif à l'organe de coupe, on lui imprime, dans une direction de coupe matérialisée 50, un mouvement relativement lent d'entraînement en rotation autour d'un axe fixe 40 et on combine à ce mouvement, dans cette même direction de coupe, un mouvement vibratoire plus rapide et de relativement faible amplitude réalisé autour d'une succession de positions occupées par l'organe de coupe, c'est-à-dire le disque denté 700, au cours de son mouvement de rotation.

On obtient ces deux mouvements combinés comme suit.

a. Mouvement oscillatoire ou vibratoire du disque.

L'engrenage 310 commandé par l'arbre 420 lié au moteur 600, fait tourner plus rapidement l'engrenage 320 qui porte l'arbre 400 muni du téton excentrique 500 autour duquel est monté le roulement à billes 910 et qui imprime à l'arbre formé en L 430 un mouvement d'oscillation relativement rapide autour de l'axe principal 40, lequel arbre transmet ce mouvement aux dents du disque 700.

b. Mouvement de rotation du disque

L'engrenage 300 également lié à l'arbre 420 commandé par le moteur 600 entraîne dans un mouvement de rotation relativement lent (par rapport au mouvement vibratoire précédent) l'arbre 430, par l'intermédiaire des engrenages réducteurs 330, 350 et 340.

Le disque 700 est donc entraîné en un mouvement combiné de rotation relativement lent et d'oscillation beaucoup plus rapide dans la direction de coupe 50 et autour de l'axe fixe 40.

Comme on le comprend, l'invention ne se limite pas aux modes de réalisation qui ont été plus spécialement envisagés, mais embrasse au contraire d'éventuelles variantes qui comprendraient des moyens équivalents de mise en oeuvre.

On pourrait en particulier prévoir pour assurer le mouvement vibratoire ou oscillatoire de l'organe de coupe, un système à came ou encore un vibreur ou un dispositif électro-pneumatique, venant en substitution ou en complément du téton excentrique.

**Revendications**

1. Procédé pour couper des corps ou des matériaux au moyen d'un organe de coupe du type lame de scie (3, 700), dans lequel
- on imprime à ladite lame, d'une part un premier mouvement périodique de relativement grande période orienté dans une direction de coupe déterminée et, d'autre part, un mouvement oscillatoire de plus petite période que celle dudit premier mouvement périodique,
- et on combine ces deux mouvements
ledit procédé étant caractérisé en ce que, de façon à couper des corps ou des matériaux relativement durs sans couper d'autres corps ou matériaux plus tendres, on imprime ledit mouvement oscillatoire dans la direction de coupe dudit premier mouvement périodique.

2. Procédé selon la revendication 1 caractérisé en ce qu'on imprime à ladite lame (3)
- d'une part un mouvement linéaire alternatif relativement lent et de relativement grande amplitude orienté dans un axe de coupe (25) déterminé,
- et, d'autre part, dirigé dans ce même axe (25) de coupe, un mouvement oscillatoire plus rapide et de plus faible amplitude que celle dudit mouvement linéaire, et ce autour d'une succession de positions occupées par la lame (3) au cours de son mouvement linéaire alternatif.

3. Procédé selon la revendication 1 caractérisé en ce qu'on imprime à ladite lame (700)
- d'une part un mouvement relativement lent d'entraînement en rotation dans une direction et autour d'un axe fixe (40) déterminés,
- et d'autre part, dans cette même direction et autour de ce même axe (40), un mouvement oscillatoire de relativement faible amplitude, plus rapide que le mouvement d'entraînement en rotation précité, et ce autour d'une succession de positions occupées par la lame (700) au cours de son mouvement de rotation.

4. Appareil de coupe du type scie sauteuse pour la mise en oeuvre du procédé selon la revendication 1 ou la revendication 2 comprenant
- un boîtier comportant une partie fixe (2) et une partie mobile (31)
- une lame de coupe (3) qui se prolonge dans le bâti par une tige (15) de commande montée mobile en translation par rapport à ladite partie fixe (2) du bâti par l'intermédiaire de moyens d'entraînement liés à ladite partie mobile (31), cesdits moyens comprenant:
- un moteur (4) de commande en rotation d'un premier arbre (14) caractérisé en ce qu'il comprend
- une série d'engrenages (8, 6, 7, 5) en prise sur ledit premier arbre (14) et formant moyens de transmission dudit mouvement de rotation à un second arbre (16) sur lequel est calé au moins un premier excentrique (9, 19 ; 9', 19') de liaison à ladite partie fixe (2) du bâti, par rapport à laquelle le premier excentrique est monté libre en rotation, ledit second arbre (16) étant lié mécaniquement à ladite tige (15) de sort à l'entraîner dans un mouvement linéaire alternatif axial relativement lent et de relativement grande amplitude, et
- un second excentrique (10) calé sur ledit premier arbre (14) et lié mécaniquement à ladite tige (15) de sorte à l'entraîner, au cours de son dit mouvement linéaire alternatif axial, dans un second mouvement oscillatoire dirigé dans le même axe (25).

5. Appareil de coupe du type scie circulaire pour la mise en oeuvre du procédé selon la revendication 1 ou la revendication 3, comprenant
- un boîtier (200)
- une lame de coupe (700) extérieure au boîtier et montée mobile par rapport à lui suivant une direction de coupe déterminée par l'intermédiaire de moyens d'entraînement comprenant :
- un moteur (600) de commande en rotation d'un premier arbre (420) caractérisé en ce qu'il comprend
- une série d'engrenages (300, 330, 310, 320, 340, 350) en prise sur ledit premier arbre (420) et formant moyens de transmission du mouvement de rotation à un deuxième arbre (400),
- un troisième arbre (430) lié mécaniquement d'un côté audit deuxième arbre (400) et, de l'autre, à la lame (700) au niveau d'une partie qu'il présente faisant saillie vers l'extérieur du boîtier (200), de sorte à entraîner ladite lame en rotation autour d'un axe (40), et
- un excentrique (500) calé sur ledit deuxième arbre (400) et formant moyen de liaison mécanique entre ce même deuxième arbre et ledit troisième arbre (430) par rapport auquel il est monté libre en rotation, de sorte que ladite lame soit, au cours de son mouvement de rotation précité, entraînée dans un mouvement oscillatoire dirigé dans la direction de coupe.

6. Appareil selon la revendication 5 caractérisé en ce que ledit troisième arbre (430) présente, au niveau de sa liaison avec l'excentrique (500), une forme en fourchette à double dents (451, 452) entre lesquelles est logé et vient pivoter ce même excentrique (500).

**Patentansprüche**

1. Verfahren zum Schneiden von Körpern oder Materialien mittels eines Schneidorganes mit einem Sägeblatt (3, 700),
- wobei man dem Blatt einerseits eine periodische erste Bewegung mit relativ großer Periode gibt, die in einer bestimmten Schneidrichtung ausgerichtet ist, und andererseits eine Oszillationsbewegung mit kleinerer Periode als die der ersten periodischen Bewegung gibt,
- und man diese beiden Bewegungen kombiniert, wobei dieses Verfahren dadurch gekennzeichnet ist, daß man, um relativ harte Körper oder Materialien zu schneiden, ohne andere, weichere Körper oder Materialien zu schneiden, diese oszillierende Bewegung in die Schneidrichtung der ersten periodischen Bewegung aufbringt.

2. Verfahren nach Anspruch 1, dadurch gekennzeichnet, daß man auf dieses Blatt (3)
- einerseits eine relativ langsame, lineare Wechselbewegung mit relativ großer Amplitude, die auf eine bestimmte Schneidachse (25) ausgerichtet ist,
- und andererseits, auf dieselbe Schneidachse (25) ausgerichtet, eine schnellere Oszillationsbewegung aufbringt mit kleinerer Amplitude als die der linearen Bewegung und dies um eine Folge von Positionen, welche durch das Blatt (3) im Verlaufe seiner linearen Wechselbewegung belegt werden.

3. Verfahren nach Anspruch 1, dadurch gekennzeichnet, daß man auf das Blatt (700)
- einerseits eine relativ langsame Antriebsdrehbewegung in einer Richtung und um eine bestimmte, feste Achse (40) aufbringt,
- und andererseits in derselben Richtung und um dieselbe Achse (40) eine Oszillationsbewegung mit relativ kleiner Amplitude aufbringt, die schneller ist als die erwähnte Antriebsdrehbewegung und dies um eine Folge von Stellungen, die von dem Blatt (700) im Verlaufe seiner Rotationsbewegung belegt werden.

4. Schneidvorrichtung vom Typ einer Stichsäge zur Durchführung des Verfahrens nach Anspruch 1 oder Anspruch 2, mit
- einem Gehäuse mit einem festen Teil (2) und einem beweglichen Teil (31) und
- einem Schneidblatt (3), welches sich in dem Gestell durch eine Steuerstange (15) verlängert, die translatorisch beweglich bezüglich dem festen Teil (2) des Gestelles mittels Antriebsmitteln angeordnet ist, die mit dem beweglichen Teil (31) verbunden sind, wobei diese Mittel
- einen Rotationssteuermotor (4) mit einer ersten Achse (14) aufweisen, dadurch gekennzeichnet, daß sie aufweist
- eine Reihe von Zahnrädern (8, 6, 7, 5), die auf dieser ersten Welle (14) in Eingriff stehen und Transmissionsmittel für diese Drehbewegung auf eine zweite Welle (16) bilden, auf welche mindestens ein erster Exzenter (9, 19; 9', 19') zur Verbindung mit dem festen Teil (2) des Gestells festsitzt, bezüglich welchem (2) der erste Exzenter in freier Rotation angeordnet ist, wobei die zweite Welle (16) mechanisch mit der Stange (15) so verbunden ist, daß sie in einer linearen, axialen, relativ langsamen und mit relativ großer Amplitude versehenen Wechselbewegung angetrieben wird, und
- daß sie einen zweiten Exzenter (10) aufweist, der auf der ersten Welle (14) festsitzt und mechanisch mit der Stange (15) derart verbunden ist, daß diese im Verlaufe ihrer linearen, axialen Wechselbewegung in eine zweite Oszillationsbewegung mitgenommen wird, die auf dieselbe Achse (25) ausgerichtet ist.

5. Schneidvorrichtung vom Typ einer Kreissäge zur Durchführung des Verfahrens nach Anspruch 1 oder Anspruch 3, mit
- einem Gehäuse (200),
- einem Schneidblatt (700) außerhalb des Gehäuses und beweglich bezüglich diesem angeordnet, wobei das Schneidblatt einer bestimmten Schneidrichtung mittels Antriebsmitteln folgt mit:
- einem Steuerrotationsmotor (600) einer ersten Welle (420), dadurch gekennzeichnet, daß sie aufweist
- eine Reihe von Zahnrädern (300, 330, 310, 320, 340, 350) in Eingriff mit dieser ersten Welle (420), wobei die Zahnräder Transmissionsmittel für die Rotationsbewegung auf eine zweite Welle (400) bilden,
- eine dritte Welle (430), die mechanisch mit einer Seite mit dieser zweiten Welle (400) verbunden ist und mit der anderen mit dem Blatt (700) an einem Teil, den sie auskragend nach außerhalb des Gehäuses (200) derart aufweist, daß das Blatt um eine Achse (40) in Rotations angetrieben wird, und
- einen Exzenter (500), der auf dieser zweiten Welle (400) festsitzt und mechanische Verbindungsmittel bildet zwischen dieser zweiten Welle und der dritten Welle (430), bezüglich welcher er frei drehbar angeordnet ist derart, daß das Blatt im Verlaufe seiner genannten Drehbewegung in einer Oszillationsbewegung mitgenommen wird, die in der Schneidrichtung ausgerichtet ist.

6. Vorrichtung nach Anspruch 5, dadurch gekennzeichnet, daß die dritte Welle (430) an ihrer Verbindung mit dem Exzenter (500) eine Art Gabel mit zwei Zähnen (451, 452) aufweist, zwischen welchen dieser Exzenter (500) angeordnet ist und sich dreht.

**Claims**

1. A method of cutting bodies or materials with the aid of a cutting means of the saw blade type (3, 700) in which:
- on the one hand, a first periodic movement of relatively prolonged cycle and orientated in a specific cutting direction is imparted to the said blade, while on the other there is also imparted to the aforesaid blade an oscillatory movement of smaller cycle than that of the said first periodic movement,
- and these two movements are combined, the said method being characterised in that in order to cut relatively hard bodies or materials without cutting other softer bodies or materials, the said oscillatory movement is imposed in the cutting direction of the said first periodic movement.

2. A method according to claim 1, characterised in that the following are imposed on the said blade (3)
- firstly an alternating relatively slow linear movement of relatively wide amplitude, orientated in a specific cutting axis (25),
- and, on the other, directed in this same cutting axis (25), a faster oscillatory movement of a smaller amplitude than that of the said linear movement, doing so about a succession of positions occupied by the blade (3) during the course of its alternating linear movement.

3. A method according to claim 1, characterised in that it imposes on the said blade (700)
- firstly a relatively slow rotatingly driving movement in a specific direction and about a specific fixed axis (40),
- and, on the other, in this same direction and about this same axis (40) an oscillatory movement of relatively small amplitude which is faster than the aforesaid rotating driving movement, doing so about a succession of positions occupied by the blade (700) during the course of its rotary movement.

4. A cutting apparatus of the pad saw type for carrying out the method according to claim 1 or claim 2, comprising
- a casing comprising a fixed part (2) and a movable part (31).
- a cutting blade (3) which is extended into the frame by a control rod (15) mounted for translatory movement in relation to the said fixed part (2) of the frame via drive means connected to the said movable part (31), the said means comprising:
- a motor (4) for controlling the rotary movement of a first shaft (14) comprising
- a series of gears (8, 6, 7, 5) meshing with the first shaft (14) and forming means of transmitting the said rotary movement to a second shaft (16) on which there is keyed at least one first eccentric member (9, 19; 9', 19') for connection to the said fixed part (2) of the frame, in respect of which the first eccentric member is mounted for free rotation, the second shaft (16) being mechanically connected to the said rod (15) in such a way as to drive it with a relatively slow alternating axial linear movement of relatively wide amplitude, and
- a second eccentric member (10) keyed on the said first shaft (14) and mechanically connected to the said rod (15) in such a way as to drive it, during the course of its said alternating axial linear movement, in a second oscillatory movement which is directed in the same axis (25).

5. A cutting apparatus of the circular saw type for carrying out the method according to claim 1 or claim 3, comprising
- a casing (200)
- a cutting blade (700) outside the casing and mounted for movement in relation to it in a cutting direction which is determined by drive means comprising:
- a motor (600) for rotating a first shaft (420), comprising
- a series of gears (300, 330, 310, 320, 340, 350) meshing with the said first shaft (420) and forming means of transmitting the rotary movement to a second shaft (400),
- a third shaft (430) connected mechanically on the one hand to the said second shaft (400) and, on the other, to the e blade (700) at the level of a part of it which projects outwardly from the casing (200) in such a way as to drive the said blade, causing it to rotate about an axis (40), and
- an eccentric member (500) keyed on the said second shaft (400) and forming a mechanical connecting means between the said second shaft and the said third shaft (430) in respect of which it is mounted for free rotation so that the said blade, during the course of its aforesaid rotary movement, is driven to perform an oscillatory movement which is directed in the cutting direction.

6. An apparatus according to claim 5, characterised in that, at the level at which it is connected to the eccentric member (500), the said third shaft (430) is in the shape of a fork with double teeth (451, 452) between which this same eccentric member (500) is held and pivoted.

FIG_1

FIG_2

EP 0 258 136 B1

FIG_4

FIG_3

EP 0 258 136 B1